# EUROPEAN PATENT APPLICATION

(11) **EP 4 130 020 A1**
(43) Date of publication of application: **08.02.2023**
(21) Application number: 21776573.4
(22) Date of filing: 24.03.2021
(51) Int. Cl.: C07J 75/00, C07J 13/00, C07J 5/00, C07J 9/00

(54) **NOVEL METHOD FOR PREPARING INOTODIOL**

(30) Priority: 26.03.2020 KR 20200036790; 21.07.2020 KR 20200090507
(71) Applicant: Daegu-Gyeongbuk Medical Innovation Foundation, Daegu 41061 (KR)
(72) Inventor: KIM, Soong-Hyun, Daegu 41061 (KR); KO, Eun Bi, Daegu 41061 (KR); SONG, Minsoo, Daegu 41061 (KR); PARK, Ga Young, Daegu 41061 (KR); LEE, Eunhye, Daegu 41061 (KR); BAE, Seri, Daegu 41061 (KR); KANG, Jihee, Daegu 41061 (KR); PARK, Yoojin, Daegu 41061 (KR); CHOI, Youjeong, Daegu 41061 (KR)
(74) Representative: Boult Wade Tennant LLP
(86) International application number: PCT/KR2021/003622
(87) International publication number: WO 2021/194244

(57) **Abstract**

The present invention relates to a novel method for preparing inotodiol, which is different from the existing method for preparing inotodiol and has a high yield of inotodiol, so it can be effectively used for preparing and mass-producing inotodiol.

## Description

### BACKGROUND OF THE INVENTION

### 1. Field of the Invention

The present invention relates to a novel method for preparing inotodiol.

### 2. Description of the Related Art

Inotodiol is triterpenoid diol derived from Inonotus obliquus, which is known to have an anticancer effect, and is a substance that has been actively studied in Russia and Finland.

Non-patent reference 1 discloses a method of obtaining inotodiol according to the following process. First, lanosterol is converted into tetranor-22-olefin through three steps, which includes a process of decarboxylation using cupric acetate. Thereafter, the side chain double bond is directly epoxidized using peracid, and an excess of isobutenyl Grignard reagent is treated to obtain inotodiol. However, the non-patent reference 1 discloses that when an epoxide intermediate is generated, a by-product having a different absolute configuration may be formed, and accordingly, an isomer having a different double bond position, not inotodiol, may be obtained as a final product.

Non-patent reference 2 discloses a method of obtaining inotodiol according to the following process. First, (22E)-3β acetoxylanosta-8,22-dien-24-one is prepared from lanosterol, which is then treated with H₂O₂ to obtain epoxide. After treatment with hydrazine hydrate, acetylation is induced, and inotodiol is obtained by treatment with sodium borohydride and phosphoryl chloride. However, the non-patent document 2 discloses that when an epoxide intermediate is generated, a by-product having a different absolute configuration may be formed, and accordingly, an isomer having a different absolute configuration may be obtained as a final product.

As described above, in the conventional method for preparing inotodiol, a manufacturing process is complicated and the yield of the target inotodiol is low due to the by-products obtained, and thus the present inventors have studied to provide a novel method for preparing inotodiol. As a result, the present inventors have found a preparation method that has a high yield of inotodiol and differs from the conventional method for preparing inotodiol in each step of the manufacturing process.

### [PRIOR ART REFERENCE]

### [NON-PATENT REFERENCE]

(Non-Patent Reference 1) Tetrahedron Volume 30, Issue 8, 1974, Pages 977-986.
(Non-Patent Reference 2) Chem. Pharm. Bull. 31(3) 907-911 (1983).

### SUMMARY OF THE INVENTION

It is an object of the present invention to provide a novel method for preparing inotodiol.

It is another object of the present invention to provide an intermediate compound of the above preparation method, an isomer thereof, a solvate thereof, or a salt thereof.

To achieve the above objects, in one aspect of the present invention, the present invention provides a method for preparing a compound represented by formula 1 comprising the following steps, as shown in reaction formula 1 below:
step 1 of preparing a compound represented by formula 9 by epoxization of a compound represented by formula 8;
step 2 of preparing a compound represented by formula 10 by reacting the compound represented by formula 9;
step 3 of preparing a compound represented by formula 11 by reacting the compound represented by formula 10; and
step 4 of preparing a compound represented by formula 1 by reacting the compound represented by formula 11:

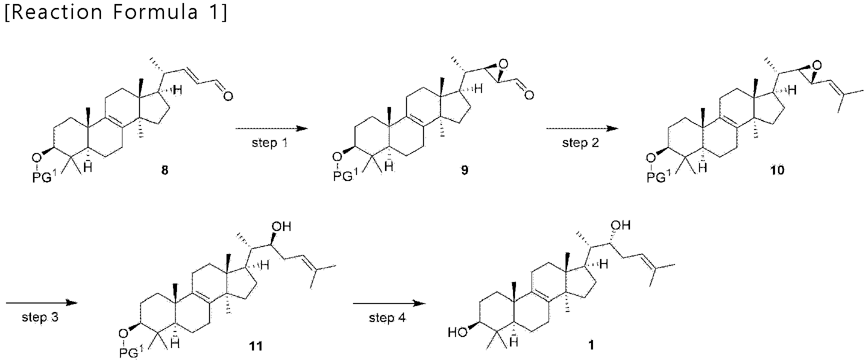

In reaction formula 1 above,
PG¹ is a protecting group of an alcohol.

In another aspect of the present invention, the present invention provides a compound represented by formula 8, an isomer thereof, a solvate thereof or a salt thereof.

In another aspect of the present invention, the present invention provides a compound represented by formula 11, an isomer thereof, a solvate thereof or a salt thereof.

In another aspect of the present invention, the present invention provides a compound represented by formula 10, an isomer thereof, a solvate thereof or a salt thereof.

In another aspect of the present invention, the present invention provides a compound represented by formula 9, an isomer thereof, a solvate thereof or a salt thereof.

In another aspect of the present invention, the present invention provides a compound represented by formula 7, an isomer thereof, a solvate thereof or a salt thereof.

In another aspect of the present invention, the present invention provides a compound represented by formula 3', an isomer thereof, a solvate thereof or a salt thereof.

In another aspect of the present invention, the present invention provides a compound represented by formula 4', an isomer thereof, a solvate thereof or a salt thereof.

### ADVANTAGEOUS EFFECT

In the method for preparing inotodiol provided in one aspect of the present invention, compared to the conventional method for preparing inotodiol, the intermediate structure and process are different as well as the process steps can be significantly reduced and the yield is high. Therefore, the method of the present invention has an effect that can be usefully used for the preparation and mass production of inotodiol.

### BRIEF DESCRIPTION OF THE DRAWINGS

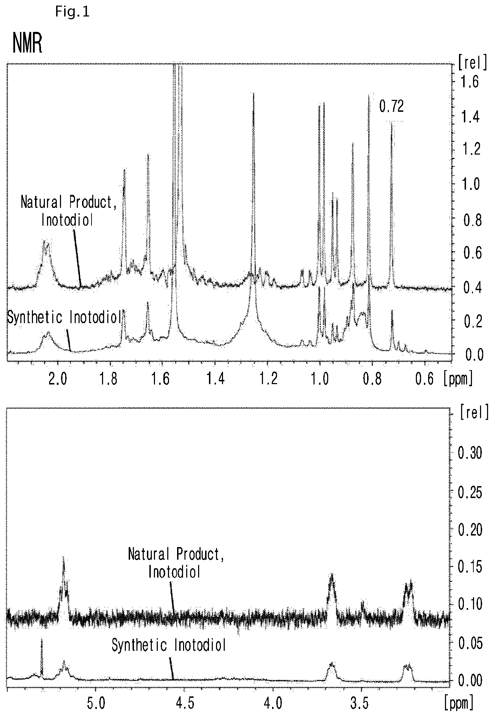

Figure 1 is NMR analysis data of the natural product inotodiol, and the inotodiol obtained according to the preparation method of the present invention.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In one aspect of the present invention, the present invention provides a method for preparing a compound represented by formula 1 comprising the following steps, as shown in reaction formula 1 below:
step 1 of preparing a compound represented by formula 9 by epoxization of a compound represented by formula 8;
step 2 of preparing a compound represented by formula 10 by reacting the compound represented by formula 9;
step 3 of preparing a compound represented by formula 11 by reacting the compound represented by formula 10; and
step 4 of preparing a compound represented by formula 1 by reacting the compound represented by formula 11:

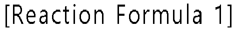

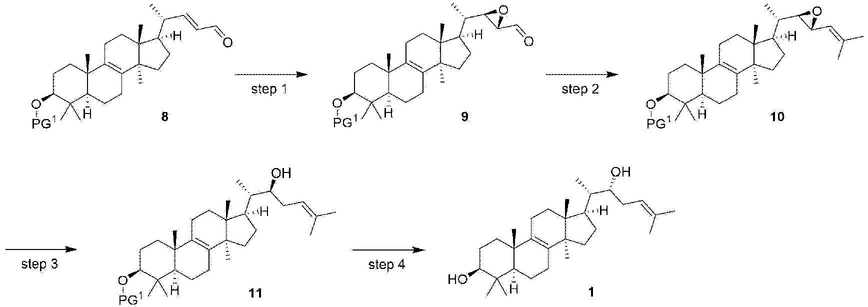

In reaction formula 1 above,
PG¹ is a protecting group of an alcohol.

In reaction formula 1 above,
PG¹ can be a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2-(trimethylsilyl)ethoxymethyl (SEM) and (phenyldimethylsilyl)methoxymethyl (SMOM).

In the preparation method represented by the reaction formula 1 according to the present invention, step 1 is a step of preparing a compound represented by formula 9 by epoxization of a compound represented by formula 8. Specifically, this step is a step of preparing a compound represented by formula 9 by epoxization of a compound represented by formula 8 in the presence of a peroxide, an organic amine, and a solvent.

In step 1, chloral hydrate can be used as a phase transfer catalyst in order to facilitate the movement of hydrogen peroxide in the organic solvent and promote the reaction. As the phase transfer catalyst, the compound 9 itself can be used, or tetrabutylammonium chloride (n-Bu₄N⁺Cl⁻), acetone, chloroacetone, 1,3-dichloroacetone, 1,1,1-trichloroacetone, chloral hydrate and the like can be used, and preferably chloral hydrate can be used, but this is only an example and is not limited thereto.

As the peroxide, m-CPBA, benzoyl peroxide, hydrogen peroxide, t-butyl hydroperoxide and the like can be used, and preferably hydrogen peroxide can be used, but this is only an example and is not limited thereto.

As the organic amine, pyrrolidine, various chiral proline-based organic amines and the like can be used in equivalent or catalytic amounts, but not always limited thereto.

As the solvent, tetrahydrofuran (THF), water, dimethoxyethane (DME), dimethyl sulfoxide (DMSO), *N,N*-dimethylformamide (DMF), acetonitrile (ACN), dichloromethane (DCM) and the like can be used alone or in combination, but not always limited thereto.

In addition, the reaction temperature of step 1 can be 0°C to 35°C, but not always limited thereto, and the reaction temperature can be appropriately adjusted according to the degree of progression of the reaction.

Furthermore, the reaction time of step 1 can be 10 minutes to 24 hours, but not always limited thereto, and the reaction time can be appropriately adjusted according to the degree of progression of the reaction.

In the preparation method represented by the reaction formula 1 according to the present invention, step 2 is a step of preparing a compound represented by formula 10 by reacting the compound represented by formula 9. Specifically, this step is a step of preparing a compound represented by formula 10 by olefination reaction of the compound represented by formula 9 obtained in step 1 above in the presence of an olefination reagent, a solvent, and a base.

As the olefination reagent, 5-(isopropylsulfonyl)-1-1phenyl-1H-tetrazole, 1-(isopropylsulfonyl)phenyl, 1-(isopropylsulfonyl)-3,5-di(trifluoromethyl)phenyl , 2-(isopropylsulfonyl)-1-1methyl-1H-benzoimidazole, 2-(isopropylsulfonyl)-benzothiazole, 2-(isopropylsulfonyl)-1-pyridine, 5-(Isopropylsulfonyl)-1-1 tertbutyl-1H-tetrazole, isopropyltriphenylphosphonium bromide (i-PrP⁺Ph₃Br⁻), isopropyltertbutyldiphenylsilane (i-PrSiPh₂t-Bu), and the like can be used, but not always limited thereto.

As the base, t-BuOK (potassium tert-butoxide), n-BuLi (n-butyl lithium), sec-BuLi (sec-butyl lithium), t-BuLi (t-butyl lithium), LDA (lithium diisopylamide), LiHMDS (lithium bis(trimethylsilyl)amide), NaHMDS (sodium bis(trimethylsilyl)amide), KHMDS (potassium bis(trimethylsilyl)amide), and the like can be used alone or in combination, but not always limited thereto.

As the solvent, diethyl ether, acetonitrile (ACN), benzene, toluene, and the like can be used alone or in combination, but not always limited thereto.

In addition, the reaction temperature of step 2 can be -100°C to -50°C or 0°C to 35°C, but not always limited thereto, and the reaction temperature can be appropriately adjusted according to the degree of progression of the reaction.

Furthermore, the reaction time of step 2 can be 10 minutes to 24 hours, but not always limited thereto, and the reaction time can be appropriately adjusted according to the degree of progression of the reaction.

In the preparation method represented by the reaction formula 1 according to the present invention, step 3 is a step of preparing a compound represented by formula 11 by reacting the compound represented by formula 10. Specifically, this step is a step of preparing a compound represented by formula 11 by regioselectively reducing the compound represented by formula 10 obtained in step 2 above in the presence of a hydrogen anion reagent, a salt, and a solvent.

As the hydrogen anion reagent, NaBH₃CN, LiBH₃CN, NaBH₄, LiAlH₄, NaBH(OAc)₃, DIBAL, Red-AI, L-Selectride, K-Selectride, N-Selectride, LS-Selectride, KS-Selectride, Super-Hydride and the like can be used, but not always limited thereto.

As the salt, ZnCl₂, ZnI₂ and the like can be used alone or in combination, but not always limited thereto.

As the solvent, tetrahydrofuran (THF), water, dimethoxyethane (DME), dimethyl sulfoxide (DMSO), *N,N*-dimethylformamide (DMF), acetonitrile (ACN), dichloromethane (DCM) and the like can be used alone or in combination, but not always limited thereto.

In addition, the reaction temperature of step 3 can be 0°C to 35°C, but not always limited thereto, and the reaction temperature can be appropriately adjusted according to the degree of progression of the reaction.

Furthermore, the reaction time of step 3 can be 10 minutes to 5 days, but not always limited thereto, and the reaction time can be appropriately adjusted according to the degree of progression of the reaction.

In the preparation method represented by the reaction formula 1 according to the present invention, step 4 is a step of preparing a compound represented by formula 1 by reacting the compound represented by formula 11. Specifically, this step is a step of preparing a compound represented by formula 1 by changing the stereochemistry of a hydroxyl group of the compound represented by formula 11 obtained in step 3 above through substitution reaction and simultaneously hydrolysis reaction.

Through the substitution reaction, the hydroxyl of the compound represented by formula 11 reacts with MsCI, TsCI, TfCl, and the like, which are electrophiles having a good leaving group, and is converted into -OMs, -OTs, and -OTf, which are prone to substitution reaction. Then, the superoxide nucleophile freed by 18-crown-6, a phase transfer catalyst, undergoes a substitution reaction to change the stereochemistry of the hydroxyl group, and at the same time, the protecting group of an alcohol is deprotected by hydrolysis to prepare a compound represented by formula 1 (inotodiol).

The phase transfer catalyst serves to increase the solubility of superoxide (O₂⁻), and the superoxide may act as a better oxygen nucleophile than a hydroxy group such as LiOH, NaOH, KOH, HOOH, or the like. In addition, the phase transfer catalyst induces a substitution reaction to change the stereochemistry of the hydroxyl group by removing good leaving groups such as -OMs, -OTs, -OTf, and the like, and simultaneously removes the acyl protecting group of an alcohol by hydrolysis. As the oxygen nucleophile, KO₂ can be used, and general oxygen nucleophiles such as LiOH, NaOH, KOH, HOOH, tBuOOH, and the like can be used, but not always limited thereto.

In the substitution reaction, the reaction temperature can be -20°C to 35°C, but not always limited thereto, and the reaction temperature can be appropriately adjusted according to the degree of progression of the reaction. In addition, the reaction time can be 10 minutes to 4 days, but not always limited thereto, and the reaction time can be appropriately adjusted according to the degree of progression of the reaction.

As the phase transfer catalyst, 18-crown-6, 6-crown-2, 9-crown-3, 12-crown-4, 15-crown-5, 21-crown-7, and the like can be used, but not always limited thereto.

As the solvent, tetrahydrofuran (THF), water, dimethoxyethane (DME), dimethyl sulfoxide (DMSO), *N,N*-dimethylformamide (DMF), acetonitrile (ACN), dichloromethane (DCM) and the like can be used alone or in combination, but not always limited thereto.

In addition, the reaction conditions of the deprotection reaction are different depending on the type of the protecting group of an alcohol, and the reaction can be carried out using the conventional deprotection reaction conditions, but this is only an example and not limited thereto.

The compound represented by formula 8 can be prepared through a preparation method comprising the following steps, as shown in reaction formula 2 below:
step 1 of preparing a compound represented by formula 7 by epoxization of a compound represented by formula 6; and
step 2 of preparing a compound represented by formula 8 by reacting the compound represented by formula 7:

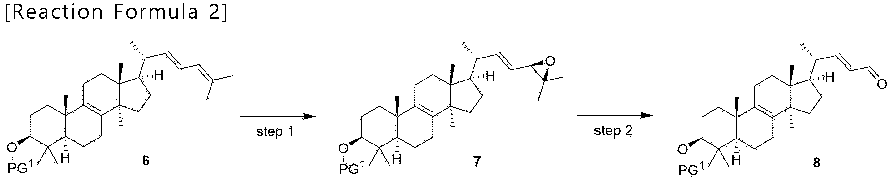

In reaction formula 2 above,
PG¹ is a protecting group of an alcohol.

In reaction formula 2 above,
PG¹ can be a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2- (trimethylsilyl)ethoxymethyl (SEM) and (phenyldimethylsilyl)methoxymethyl (SMOM).

In the preparation method represented by the reaction formula 2 according to the present invention, step 1 is a step of preparing a compound represented by formula 7 by epoxization of a compound represented by formula 6. Specifically, this step is a step of preparing a compound represented by formula 8 by epoxization of a compound represented by formula 6 in the presence of a peroxide, a salt, and a solvent.

As the peroxide, m-CPBA, benzoyl peroxide, hydrogen peroxide, t-butyl hydroperoxide and the like can be used, and preferably m-CPBA can be used, but this is only an example and is not limited thereto.

As the salt, KF, NaF, CsF, n-Bu₄NF, Me₄NF, Et₄NF, n-BuMe₃NF and the like can be used, but this is only an example and is not limited thereto.

As the solvent, tetrahydrofuran (THF), water, dimethoxyethane (DME), dimethyl sulfoxide (DMSO), *N,N*-dimethylformamide (DMF), acetonitrile (ACN), dichloromethane (DCM) and the like can be used alone or in combination, but not always limited thereto.

In addition, the reaction temperature of step 1 can be 0°C to 35°C, but not always limited thereto, and the reaction temperature can be appropriately adjusted according to the degree of progression of the reaction.

Furthermore, the reaction time of step 1 can be 10 minutes to 24 hours, but not always limited thereto, and the reaction time can be appropriately adjusted according to the degree of progression of the reaction.

In the preparation method represented by the reaction formula 2 according to the present invention, step 2 is a step of preparing a compound represented by formula 8 by reacting the compound represented by formula 7. Specifically, this step is a step of preparing a compound represented by formula 8 by periodic acid oxidation of the compound represented by formula 7 obtained in step 1 above in the presence of a periodic acid and a solvent.

As the periodic acid, HIO₄ or H₅IO₆ can be used, but not always limited thereto.

As the solvent, diethyl ether, acetonitrile (ACN), benzene, toluene and the like can be used alone or in combination, but not always limited thereto.

In addition, the reaction temperature of step 2 can be 0°C to 35°C, but not always limited thereto, and the reaction temperature can be appropriately adjusted according to the degree of progression of the reaction.

Furthermore, the reaction time of step 2 can be 10 minutes to 24 hours, but not always limited thereto, and the reaction time can be appropriately adjusted according to the degree of progression of the reaction.

The compound represented by formula 6 can be prepared through a preparation method comprising the following steps, as shown in reaction formula 3 below:
step 1 of preparing a compound represented by formula 3 by reacting a compound represented by formula 2;
step 2 of preparing a compound represented by formula 4 by elimination reaction of the compound represented by formula 3;
step 3 of preparing a compound represented by formula 5 by elimination reaction of the compound represented by formula 4; and
step 4 of preparing a compound represented by formula 6 by reacting the compound represented by formula 5:

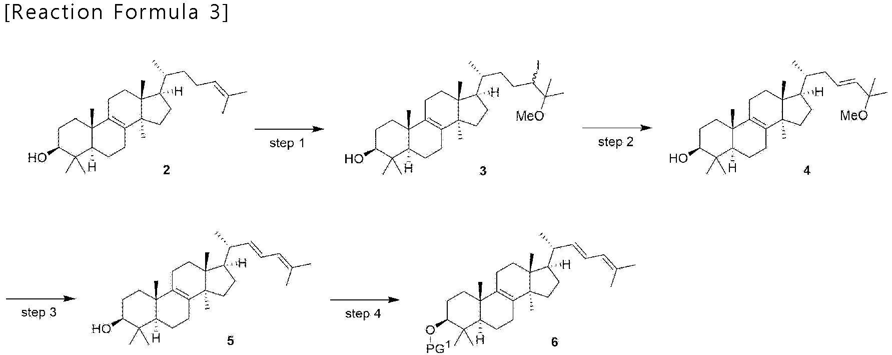

In reaction formula 3 above,
PG¹ is a protecting group of an alcohol.

In reaction formula 3 above,
PG¹ can be a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2- (trimethylsilyl)ethoxymethyl (SEM) and (phenyldimethylsilyl)methoxymethyl (SMOM).

In the preparation method represented by the reaction formula 3 according to the present invention, step 1 is a step of preparing a compound represented by formula 3 by reacting a compound represented by formula 2 with a halogen and an alcohol. Specifically, this step is a step of preparing a compound represented by formula 3 linked which a halogen and an alkoxy group by reacting a lanosterol derivative represented by formula 2 with a halogen and an alcohol in the presence of a salt and a solvent.

As the salt, Cu(OAc)₂, CuCl₂, CuBr₂, CuSO₄ and the like can be used alone or in combination, but not always limited thereto.

As the solvent, water, tetrahydrofuran (THF), dimethoxyethane (DME), dimethyl sulfoxide (DMSO), *N,N*-dimethylformamide (DMF), acetonitrile (ACN), dichloromethane and the like can be used alone or in combination, but not always limited thereto.

In addition, the reaction temperature of step 1 can be 0°C to 35°C, but not always limited thereto, and the reaction temperature can be appropriately adjusted according to the degree of progression of the reaction.

As the halogen, fluorine, chlorine, bromine or iodine can be used, and preferably iodine can be used, but this is only an example and not limited thereto.

As the alcohol, a linear or branched C₁₋₁₀ alcohol can be used, and preferably methanol can be used, but this is only an example and not limited thereto.

In the preparation method represented by the reaction formula 3 according to the present invention, step 2 is a step of preparing a compound represented by formula 4 by elimination reaction of the compound represented by formula 3. Specifically, this step is a step of preparing a compound represented by formula 4 having a double bond with a halogen removed by elimination reaction of the compound represented by formula 3 obtained in step 1 above in the presence of a base and a solvent.

As the base, t-BuOK (potassium tert-butoxide), LiHMDS (lithium bis(trimethylsilyl)amide), NaHMDS (sodium bis(trimethylsilyl)amide), KHMDS (potassium bis(trimethylsilyl)amide) and the like can be used alone or in combination, but not always limited thereto.

As the solvent, tetrahydrofuran (THF), water, dimethoxyethane (DME), dimethyl sulfoxide (DMSO), *N,N*-dimethylformamide (DMF), acetonitrile (ACN), dichloromethane and the like can be used alone or in combination, but not always limited thereto.

In addition, the reaction time of step 2 can be 10 minutes to 24 hours, but not always limited thereto, and the reaction time can be appropriately adjusted according to the degree of progression of the reaction.

In the preparation method represented by the reaction formula 3 according to the present invention, step 3 is a step of preparing a compound represented by formula 5 by elimination reaction of the compound represented by formula 4. Specifically, this step is a step of preparing a compound represented by formula 5 having a double bond with an alkoxy removed by elimination reaction of the compound represented by formula 4 obtained in step 2 above in the presence of a solvent.

As the solvent, tetrahydrofuran (THF), water, dimethoxyethane (DME), dimethyl sulfoxide (DMSO), *N,N*-dimethylformamide (DMF), acetonitrile (ACN), dichloromethane (DCM) and the like can be used alone or in combination, but not always limited thereto.

According to an embodiment of the present invention, AcCI can be used in step 3 above. AcCI attaches to the oxygen of -OMe and activates it as a good leaving group of -MeO⁺Ac. If there is a double bond at the beta-gamma position, the chloride anion or iodide anion of NaI removed from the front removes the proton at the delta position. While forming two double bonds at the alpha-beta and gamma-delta positions, an elimination reaction may occur in which the activated -MeO⁺Ac leaving group is separated in the form of MeOAc (methyl acetate). At this time, most silyl chloride materials such as Me₃SiCl, Et₃SiCl, tBuMe₂SiCl and the like can be used in addition to AcCI.

According to an embodiment of the present invention, NaI can be used in step 3 above, and in addition, various iodide salts such as KI and n-Bu₄NI can be used.

In addition, the reaction temperature of step 3 can be 0°C to 35°C, but not always limited thereto, and the reaction temperature can be appropriately adjusted according to the degree of progression of the reaction.

Furthermore, the reaction time of step 3 can be 10 minutes to 24 hours, but not always limited thereto, and the reaction time can be appropriately adjusted according to the degree of progression of the reaction.

In the preparation method represented by the reaction formula 3 according to the present invention, step 4 is a step of preparing a compound represented by formula 6 by reacting the compound represented by formula 5. Specifically, this step is a step of preparing a compound represented by formula 6 by an alcohol protecting group formation reaction of the compound represented by formula 5 obtained in step 3 above in the presence of a base and a solvent.

As the base, an organic amine such as DBU, pyridine, triethylamine, diisopropylethyl amine or pyrrolidine can be used alone or in combination, but not always limited thereto.

As the solvent, tetrahydrofuran (THF), water, dimethoxyethane (DME), dimethyl sulfoxide (DMSO), *N,N*-dimethylformamide (DMF), acetonitrile (ACN), dichloromethane (DCM) and the like can be used alone or in combination, but not always limited thereto.

The alcohol protecting group is a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2- (trimethyl Silyl)ethoxymethyl (SEM) and (phenyldimethylsilyl)methoxymethyl (SMOM), and is preferably acetyl (Ac). Acetic anhydride can be used to introduce acetyl (Ac) as the protecting group.

In addition, the reaction temperature of step 4 can be 0°C to 35°C, but not always limited thereto, and the reaction temperature can be appropriately adjusted according to the degree of progression of the reaction.

Furthermore, the reaction time of step 4 can be 10 minutes to 24 hours, but not always limited thereto, and the reaction time can be appropriately adjusted according to the degree of progression of the reaction.

In another aspect of the present invention, the present invention provides a compound represented by formula 8, an isomer thereof, a solvate thereof or a salt thereof.

In another aspect of the present invention, the present invention provides a compound represented by formula 11, an isomer thereof, a solvate thereof or a salt thereof.

In another aspect of the present invention, the present invention provides a compound represented by formula 10, an isomer thereof, a solvate thereof or a salt thereof.

In another aspect of the present invention, the present invention provides a compound represented by formula 9, an isomer thereof, a solvate thereof or a salt thereof.

In another aspect of the present invention, the present invention provides a compound represented by formula 7, an isomer thereof, a solvate thereof or a salt thereof.

In another aspect of the present invention, the compound represented by formula 8 can be prepared through a preparation method comprising the following steps, as shown in reaction formula 4 below:
step 1 of preparing a compound represented by formula 2' by introducing a protecting group to a compound represented by formula 1';
step 2 of preparing a compound represented by formula 3' by epoxization of the compound represented by formula 2' prepared in step 1 above;
step 3 of preparing a compound represented by formula 4' by reacting the compound represented by formula 3' prepared in step 2 above; and
step 4 of preparing a compound represented by formula 8 by reacting the compound represented by formula 4' prepared in step 3 above:

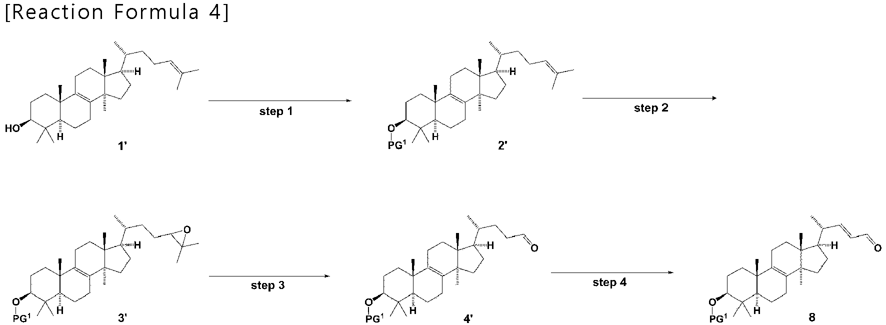

In reaction formula 4 above,
PG¹ is a protecting group of an alcohol.

In reaction formula 4 above,
PG¹ can be a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2- (trimethylsilyl)ethoxymethyl (SEM) and (phenyldimethylsilyl)methoxymethyl (SMOM).

In the preparation method represented by the reaction formula 4 according to the present invention, step 1 is a step of preparing a compound represented by formula 2' by introducing a protecting group to a compound represented by formula 1'. Specifically, this step is a step of preparing a compound represented by formula 2' by an alcohol protecting group formation reaction of a compound represented by formula 1' in the presence of a base and a solvent.

As the base, an organic amine such as diisopropylethylamine, 1,8-diazabicyclo[5,4,0]undec-7-ene (DBU), pyridine, triethylamine or pyrrolidine can be used alone or in combination, but not always limited thereto.

As the solvent, dichloromethane, tetrahydrofuran (THF), water, dimethoxyethane (DME), dimethyl sulfoxide (DMSO), *N,N*-dimethylformamide (DMF), acetonitrile (ACN) and the like can be used alone or in combination, but not always limited thereto.

The alcohol protecting group can be a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2- (trimethyl Silyl)ethoxymethyl (SEM) and (phenyldimethylsilyl)methoxymethyl (SMOM).

In an embodiment of the present invention, methoxymethyl (MOM) is used as the alcohol protecting group.

In addition, the reaction can be carried out in a temperature range of 0°C to 80°C, but the reaction temperature can be appropriately adjusted according to the degree of progression of the reaction.

Furthermore, the reaction can be carried out for 7 to 21 hours, but the reaction time can be appropriately adjusted according to the degree of progression of the reaction.

In the preparation method represented by the reaction formula 4 according to the present invention, step 2 is a step of preparing a compound represented by formula 3' by epoxization of the compound represented by formula 2' prepared in step 1 above. Specifically, this step is a step of preparing a compound represented by formula 3' by epoxization of the compound represented by formula 2' prepared in step 1 above in the presence of a peroxide and a solvent.

As the peroxide, m-CPBA, benzoyl peroxide, hydrogen peroxide, t-butyl hydroperoxide and the like can be used, and preferably m-CPBA can be used, but this is only an example and is not limited thereto.

As the solvent, dichloromethane, tetrahydrofuran (THF), water, dimethoxyethane (DME), dimethyl sulfoxide (DMSO), *N,N*-dimethylformamide (DMF), acetonitrile (ACN) and the like can be used alone or in combination, but not always limited thereto.

In addition, the reaction can be carried out in a temperature range of 0°C to 80°C, but the reaction temperature can be appropriately adjusted according to the degree of progression of the reaction.

Furthermore, the reaction can be carried out for 7 to 21 hours, but the reaction time can be appropriately adjusted according to the degree of progression of the reaction.

In the preparation method represented by the reaction formula 4 according to the present invention, step 3 is a step of preparing a compound represented by formula 4' by reacting the compound represented by formula 3' prepared in step 2 above. Specifically, this step is a step of preparing a compound represented by formula 4' by periodic acid oxidation of the compound represented by formula 3' obtained in step 2 above in the presence of a periodic acid and a solvent.

At this time, as the periodic acid, HIO₄ or H₅IO₆ can be used, but not always limited thereto.

As the solvent, diethyl ether, acetonitrile (ACN), benzene, toluene and the like can be used alone or in combination, but not always limited thereto.

In addition, the reaction can be carried out in a temperature range of 0°C to 80°C, but the reaction temperature can be appropriately adjusted according to the degree of progression of the reaction.

Furthermore, the reaction can be carried out for 1 to 5 hours, but the reaction time can be appropriately adjusted according to the degree of progression of the reaction.

In the preparation method represented by the reaction formula 4 according to the present invention, step 4 is a step of preparing a compound represented by formula 8 by reacting the compound represented by formula 4' prepared in step 3 above. Specifically, this step is a step of preparing a compound represented by formula 8 in which two hydrogens are eliminated and a double bond is generated by treating the compound represented by formula 4' prepared in step 3 above with 2-iodoxybenzoic acid (IBX) and trifluoroacetic acid (TFA).

At this time, dimethyl sulfoxide (DMSO), dichloromethane, tetrahydrofuran (THF), water, dimethoxyethane (DME), *N,N*-dimethylformamide (DMF), acetonitrile (ACN) and the like can be used alone or in combination as the solvent, but not always limited thereto.

In addition, the reaction can be carried out in a temperature range of 0°C to 80°C, but the reaction temperature can be appropriately adjusted according to the degree of progression of the reaction.

Furthermore, the reaction can be carried out for 5 hours to 12 days, but the reaction time can be appropriately adjusted according to the degree of progression of the reaction.

The compound represented by Formula 8 prepared in the above step is a precursor for inotodiol synthesis having a novel structure, and is used for inotodiol synthesis, thereby can significantly increase the overall synthesis efficiency of inotodiol.

In another aspect of the present invention, the present invention provides a compound represented by formula 3', an isomer thereof, a solvate thereof or a salt thereof.

In another aspect of the present invention, the present invention provides a compound represented by formula 4', an isomer thereof, a solvate thereof or a salt thereof.

At this time, the isomer can be a stereoisomer, and more specifically, an optical isomer.

In addition, the salt can be a pharmaceutically acceptable salt.

In the method for preparing inotodiol provided in one aspect of the present invention, compared to the conventional method for preparing inotodiol, the intermediate structure and process are different as well as the process steps can be significantly reduced and the yield is high. Therefore, the method of the present invention has an effect that can be usefully used for the preparation and mass production of inotodiol. This is supported by the examples described below.

Hereinafter, the present invention will be described in detail by the following examples.

However, the following examples are only for illustrating the present invention, and the contents of the present invention are not limited thereto.

### Example 1: Preparation of inotodiol (corresponding to reaction formulas 1, 2 and 3)

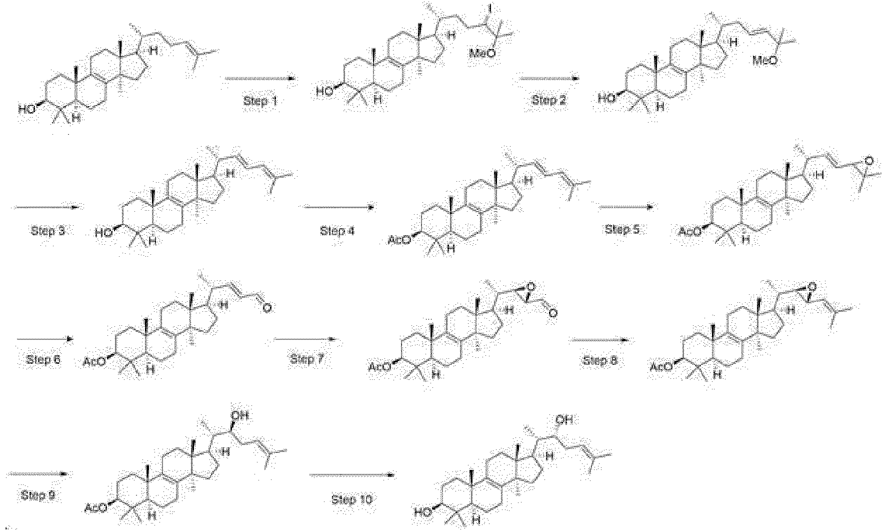

### Step 1: Preparation of (3S,5R,10S,13R,14R,17R)-17-((2R)-5-iodo-6-methoxy-6-methylheptane-2-yl)-4,4,10,13,14-pentamethyl-2,3,4,5,6,7,10,11,12,13,14,15,16,17-tetradecahydro-1H-cyclopenta[a]phenanthrene-3-ol

Lanosterol (20 g, 25.8 mmol, 1 eq, purity 55%) was dissolved in dichloromethane (200 ml), to which cupric acetate (II) monohydrate (3.60 g, 18.04 mmol, 0.7 eq) dissolved in methanol (350 ml) was added. The reactant was wrapped in aluminum foil to block light, and then iodine (6.54 g, 25.8 mmol, 1 eq) dissolved in dichloromethane (100 ml) was added thereto. The reactant was stirred at room temperature for 12 hours. Upon completion of the reaction, the solid was removed by filtration, and 30 g of potassium carbonate (K₂CO₃) in powder form was added to the filtrate. The brown reactant was stirred at room temperature for 6 hours until the color became clear. The colorless reactant was filtered, and the solvent was removed under reduced pressure. Then, the (3S,5R,10S,13R,14R,17R)-17-((2R)-5-iodo-6-methoxy-6-methylheptane-2-yl)-4,4,10,13,14-pentamethyl-2,3,4,5,6,7,10,11,12,13,14,15,16,17-tetradecahydro-1H-cyclopenta[a]phenanthrene-3-ol was obtained without further purification.

¹H NMR (400 MHz, CDCl₃) : δ4.11 - 4.03 (m, 1H), 3.29 - 3.19 (m, 4H), 1.36 (s, 6H), 1.00 (s, 3H), 0.98 (s, 3H), 0.93 (d, *J* = 6.6 Hz, 3H), 0.88 (s, 3H), 0.81 (s, 3H), 0.69 (s, 3H).

Mass (GCMS) : 584 (M⁺).

### Step 2: Preparation of (3S,5R,10S,13R,14R,17R)-17-((R,E)-6-methoxy-6-methylhept-4-ene-2-yl)-4,4,10,13,14-pentamethyl-2,3,4,5,6,7,10,11,12,13,14,15,16,17-tetradecahydro-1H-cyclopenta[a]phenanthrene-3-ol

The 3S,5R,10S,13R,14R,17R)-17-((2R)-5-iodo-6-methoxy-6-methylheptane-2-yl)-4,4,10,1 3,14-penta methyl-2, 3,4, 5,6,7,10,1 1,12,1 3,14,15,16,17-tetrad eca hyd ro-1H-cyclopenta[a]phenanthrene-3-ol (25.8 mmol, 1 eq) synthesized in step 1 was dissolved in tetrahydrofuran (THF) (450 ml), and potassium tert-butoxide (t-BuOK) (12.16 g, 108 mmol, 4.2 eq) was added thereto. The reactant was stirred under reflux in under a nitrogen stream for 4 hours. Upon completion of the reaction, the temperature was lowered to room temperature, and 10 g of ammonium chloride was added to break the excess base. The reactant was filtered, and extracted with dichloromethane after the solvent was removed under reduced pressure. The organic layer was washed sequentially with H₂O and brine, dried over anhydrous MgSO₄, and then the solvent was removed under reduced pressure. Thereafter, the reaction mixture was separated and purified by MPLC to give (3S,5R,10S,13R,14R,17R)-17-((R,E)-6-methoxy-6-methylhept-4-ene-2-yl)-4,4,10,13,14-pentamethyl-2,3,4,5,6,7,10,11,12,13,14,15,16,17-tetradecahydro-1H-cyclopenta[a]phenanthrene-3-ol (4.2 g, 9.20 mmol) in a yield of 35%.

¹H NMR (400 MHz, CDCl₃) : δ5.51 (d, *J* = 6.2 Hz, 1H), 5.39 (d, *J* = 16.3 Hz, 1H), 3.30 - 3.21 (m, 3H), 3.19 (t, *J* = 4.5 Hz, 1H), 3.15 (s, 3H), 1.26 (s, 6H), 1.00 (s, 3H), 0.98 (s, 3H), 0.90 (d, *J* = 5.8 Hz, 3H), 0.87 (d, *J* = 4.2 Hz, 3H), 0.81 (s, 3H), 0.70 (s, 3H), 0.69 (s, 3H).

Mass (GCMS) : 456 (M⁺).

### Step 3: Preparation of (3S,5R,10S,13R,14R,17R)-4,4,10,13,14-pentamethyl-17-((R,E)-6-methylhepta-3,5-diene-2-yl)-2,3,4,5,6,7,10,11,12,13,14,15,16,17-tetradecahydro-1H-cyclopenta[a]phenanthrene-3-ol

The (3S,5R,10S,13R,14R,17R)-17-((R,E)-6-methoxy-6-methylhept-4-ene-2-yl)-4,4,10,13,14-pentamethyl-2,3,4,5,6,7,10,11,12,13,14,15,16,17-tetradecahydro-1H-cyclopenta[a]phenanthrene-3-ol (4.2 g, 9.20 mmol,1 eq) synthesized in step 2 was dissolved in dichloromethane (40 ml) and acetonitrile (40 ml), to which sodium iodide (Nal) (1.38 g, 9.20 mmol, 1 eq) in powder form was added, followed by stirring under a nitrogen stream. Acetyl chloride (AcCI) (0.65 ml, 9.20 mmol, 1 eq) was added to the reactant, followed by stirring under a nitrogen stream for 15 minutes. Upon completion of the reaction, the reactant was extracted with diethyl ether, and the organic layer was washed sequentially with H₂O and brine. After drying over anhydrous MgSO₄, the solvent was removed under reduced pressure. Then, the reaction mixture was separated and purified by MPLC to give (3S,5R,10S,13R,14R,17R)-4,4,10,13,14-pentamethyl-17-((R,E)-6-methylhepta-3,5-diene-2-yl)-2,3,4,5,6,7,10,11,12,13,14,15,16,17-tetradecahydro-1H-cyclopenta[a]phenanthrene-3-ol (1.49 g, 3.51 mmol) in a yield of 38%.

¹H NMR (400 MHz, CDCl₃) : δ6.15 (dd, *J* = 14.9, 10.8 Hz, 1H), 5.76 (d, *J* = 10.9 Hz, 1H), 5.41 (dd, *J* = 14.8, 8.5 Hz, 1H), 3.24 (dd, *J* = 11.2, 4.3 Hz, 1H), 2.09 - 1.96 (m, 6H), 1.75 (s, 3H), 1.74 (s, 3H), 0.99 (s, 3H), 0.97 (s, 3H), 0.87 (s, 3H), 0.81 (s, 3H), 0.72 (s, 3H).

Mass (GCMS) : 424 (M⁺).

### Step 4: Preparation of (3S,5R,10S,13R,14R,17R)-4,4,10,13,14-pentamethyl-17-((R,E)-6-methylhepta-3,5-diene-2-yl)-2,3,4,5,6,7,10,11,12,13,14,15,16,17-tetradecahydro-1H-cyclopenta[a]phenanthrene-3-yl acetate

The (3S,5R,10S,13R,14R,17R)-4,4,10,13,14- pentamethyl-17-((R,E)-6-methylhepta-3,5-diene-2-yl)-2,3,4,5,6,7,10,11,12,13,14,15,16,17- tetradecahydro-1H-cyclopenta[a]phenanthrene-3-ol (1.49 g, 3.51 mmol, 1 eq) synthesized in step 3 was dissolved in dichloromethane (10 ml), to which 4-dimethylaminopyridine (DMAP) (0.04 g, 0.351 mmol, 0.1 eq), triethylamine (2.45 ml, 17.57 mmol, 5 eq) and acetic anhydride (0.66 ml, 7.03 mmol, 2 eq) were added. The reactant was stirred at room temperature for 1 hour. Upon completion of the reaction, methanol was added and extraction was performed with dichloromethane. The organic layer was washed sequentially with H₂O, saturated aqueous NaHCO₃ solution, and brine. After drying over anhydrous Na₂SO₄, the solvent was removed under reduced pressure. Then, the reaction mixture was separated and purified by MPLC to give (3S,5R,10S,13R,14R,17R)-4,4,10,13,14-pentamethyl-17-((R,E)-6-methylhepta-3,5-diene-2-yl)-2,3,4,5,6,7,10,11,12,13,14,15,16,17-tetradecahydro-1H-cyclopenta[a]phenanthrene-3-yl acetate (1.44 g, 3.07 mmol) in a yield of 88%.

¹H NMR (400 MHz, CDCl₃) : δ6.15 (dd, *J* = 15.0, 10.8 Hz, 1H), 5.76 (d, *J* = 10.7 Hz, 1H), 5.41 (dd, *J* = 15.0, 8.6 Hz, 1H), 4.50 (dd, *J* = 11.6, 4.5 Hz, 1H), 2.05 - 1.97 (m, 6H), 1.75(s, 3H), 1.74 (s, 3H), 1.01 (s, 3H), 0.88 (s, 6H), 0.87 (s, 3H), 0.72 (s, 3H).

Mass (GCMS) : 466 (M⁺).

### Step 5: Preparation of (3S,5R,10S,13R,14R,17R)-17-((2R,E)-4-(3,3-dimethyloxirane-2-yl)but-3-en-2-yl)-4,4,10,13,14-pentamethyl-2,3,4,5,6,7,10,11,12,13,14,15,16,17-tetradecahydro-1H-cyclopenta[a]phenanthrene-3-yl acetate

Meta-chloroperoxybenzoic acid (m-CPBA) (0.76 g, 3.07 mmol, 1 eq) and potassium fluoride (KF) (0.22 g, 3.69 mmol, 1.2 eq) were dissolved in dichloromethane (10 ml), and the reaction mixture was stirred at room temperature for 5 minutes under a nitrogen stream. The (3S,5R,10S,13R,14R,17R)-4,4,10,13,14-pentamethyl-17-((R,E)-6-methylhepta-3,5-diene-2-yl)-2,3,4,5,6,7,10,11,12,13,14,15,16,17-tetradecahydro-1H-cyclopenta[a]phenanthrene-3-yl acetate (1.44 g, 3.07 mmol,1 eq) synthesized in step 4 was dissolved in dichloromethane (10 ml), and added to the reaction mixture. The reaction mixture was stirred at room temperature for 1 hour under a nitrogen stream. Potassium fluoride (KF) (0.22 g, 3.69 mmol, 1.2 eq) was further added to the reaction mixture, and the mixture was stirred at room temperature for 1 hour under a nitrogen stream. Upon completion of the reaction, the reaction mixture was filtered to obtain a filtrate, and the solvent was removed under reduced pressure. (3S,5R,10S,13R,14R,17R)-17-((2R,E)-4-(3,3-dimethyloxirane-2-yl)but-3-en-2-yl)-4,4,10,13,14-pentamethyl-2,3,4,5,6,7,10,11,12,13,14,15,16,17-tetradecahydro-1H-cyclopenta[a]phenanthrene-3-yl acetate (1.42 g, 2.95 mmol) was obtained without a separate purification process in a yield of 96%.

¹H NMR (400 MHz, CDCl₃) δ5.76 (dd, *J* = 15.2, 8.4 Hz, 1H), 5.31 - 5.17 (m, 1H), 4.50 (dd, *J* = 11.6, 4.6 Hz, 1H), 3.15 (d, *J* = 7.2 Hz, 1H), 2.05 - 1.97 (m, 6H), 1.34 (s, 3H), 1.28 (s, 3H), 1.00 (s, 3H), 0.88 (s, 6H), 0.87 (s, 3H).

Mass (GCMS) : 482 (M⁺).

### Step 6: Preparation of (3S,5R,10S,13R,14R,17R)-4,4,10,13,14-pentamethyl-17-((R,E)-5-oxopent-3-en-2-yl)-2,3,4,5,6,7,10,11,12,1 3,14,15,16,17-tetradecahydro-1H-cyclopenta[a]phenanthrene-3-yl acetate

The (3S,5R,10S,13R,14R,17R)-17-((2R,E)-4-(3,3-dimethyloxirane-2-yl)but-3-en-2-yl)-4,4,10,13,14-pentamethyl-2,3,4,5,6,7,10,11,12,13,14,15,16,17-tetradecahydro-1H-cyclopenta[a]phenanthrene-3-yl acetate (1.42 g, 2.95 mmol) synthesized in step 5 was dissolved in diethyl ether (10 ml), to which periodic acid (2.02 g, 8.85 mmol, 3 eq) was added. The reaction mixture was stirred at room temperature for 3 hours under a nitrogen stream. Upon completion of the reaction, extraction was performed with diethyl ether. The organic layer was washed with H₂O. After drying over anhydrous MgSO₄, the solvent was removed under reduced pressure. Then, the reaction mixture was separated and purified by MPLC to give (3S,5R,10S,13R,14R,17R)-4,4,10,13,14-pentamethyl-17-((R,E)-5-oxopent-3-en-2-yl)-2,3,4,5,6,7,10,11,12,13,14,15,16,17-tetradecahydro-1H-cyclopenta[a]phenanthrene-3-yl acetate (543 mg, 1.32 mmol) in a yield of 42%.

¹H NMR (400 MHz, CDCl₃) : δ9.49 (d, *J* = 7.9 Hz, 1H), 6.72 (dd, *J* = 15.6, 8.7 Hz, 1H), 6.06 (dd, *J* = 15.4, 7.9 Hz, 1H), 4.50 (dd, *J* = 11.7, 4.5 Hz, 1H), 2.06 - 2.01 (m, 6H), 1.13 (s, 3H), 1.11 (s, 3H), 1.01 (s, 3H), 0.89 (s, 6H), 0.75 (s, 3H).

Mass (GCMS) : 440 (M⁺).

### Step 7: Preparation of (3S,5R,10S,13R,14R,17R)-17-((1S)-1-(3-formyloxirane-2-yl)ethyl)-4,4,10,13,14-pentamethyl-2,3,4,5,6,7,10,11,12,13,14,15,16,17-tetradecahydro-1H-cyclopenta[a]phenanthrene-3-yl acetate

The (3S,5R,10S,13R,14R,17R)-4,4,10,13,14-pentamethyl-17-((R,E)-5-oxopent-3-en-2-yl)-2,3,4,5,6,7,10,11,12,13,14,15,16,17-tetradecahydro-1H-cyclopenta[a]phenanthrene-3-yl acetate (543 mg, 1.32 mmol, 1 eq) synthesized in step 6 was dissolved in dichloromethane (5 ml), to which pyrrolidine (0.10 ml, 1.23 mmol, 1 eq) was added. After chloral hydrate (20.38 mg, 0.123 mmol, 0.1 eq) was added to the reaction mixture, 30 wt% of hydrogen peroxide (0.25 ml, 2.464 mmol, 2 eq) was added thereto. The reaction mixture was stirred at room temperature for 3 hours. Upon completion of the reaction, extraction was performed with dichloromethane, and the organic layer was washed sequentially with H₂O and brine, and dried over anhydrous Na₂SO₄. The solvent was removed under reduced pressure. Then, the reaction mixture was separated and purified by MPLC to give (3S,5R,10S,13R,14R,17R)-17-((1S)-1-(3-formyloxirane-2-yl)ethyl)-4,4,10,13,14-pentamethyl-2,3,4,5,6,7,10,11,12,13,14,15,16,17-tetradecahydro-1H-cyclopenta[a]phenanthrene-3-yl acetate (237 mg, 0.519 mmol) in a yield of 42%.

¹H NMR (400 MHz, CDCl₃) : δ9.01 (d, *J* = 6.3 Hz, 1H), 4.52 (d, *J* = 4.5 Hz, 1H), 3.13 - 3.04 (m, 2H), 2.06 - 2.01 (m, 6H), 1.00 (s, 3H),0.97 (d, *J* = 6.6 Hz, 3H), 0.91 (s, 3H), 0.88 (s, 6H), 0.69 (s, 3H).

Mass (GCMS) : 456 (M⁺).

### Step 8: Preparation of (3S,5R,10S,13R,14R,17R)-4,4,10,13,14-pentamethyl-17-((1S)-1-(3-(2-methylprop-1-en-1-yl)oxirane-2-yl)ethyl)-2,3,4,5,6,7,10,11,12,13,14,15,16,17-tetradecahydro-1H-cyclopenta[a]phenanthrene-3-yl acetate

The (3S,5R,10S,13R,14R,17R)-17-((1S)-1-(3-formyloxirane-2-yl)ethyl)-4,4,10,13,14-pentamethyl-2,3,4,5,6,7,10,11,12,13,14,15,16,17-tetradecahydro-1H-cyclopenta[a]phenanthrene-3-yl acetate (237 mg, 0.519 mmol, 1 eq) synthesized in step 7 and 5-(isopropylsulfonyl)-1-phenyl-1H-tetrazole (157 mg, 0.623 mmol, 1.2 eq) were dissolved in toluene (2 ml), to which potassium bis(trimethylsilyl)amide (KHMDS) (0.5 M toluene solution) (1.2 ml, 0.623 mmol, 1.2 eq) was added. The reaction mixture was stirred at -78°C for 1 hour. The reaction mixture was warmed to room temperature, to which 5-(isopropylsulfonyl)-1-phenyl-1H-tetrazole (157 mg, 0.623 mmol, 1.2 eq) and potassium bis(trimethylsilyl)amide (KHMDS) (0.5 M toluene solution) (1.2 ml, 0.623 mmol, 1.2 eq) were added. The reaction mixture was stirred at room temperature for 1 hour. 5-(Isopropylsulfonyl)-1-phenyl-1H-tetrazole (157 mg, 0.623 mmol, 1.2 eq) and potassium bis(trimethylsilyl)amide (KHMDS) (0.5 M toluene solution) (1.2 ml, 0.623 mmol, 1.2 eq) were added to the reaction mixture. The reaction mixture was stirred at room temperature for 1 hour. Upon completion of the reaction, the reaction mixture was neutralized using a supersaturated aqueous ammonium chloride solution and extracted with diethyl ether. After drying over anhydrous MgSO₄, the solvent was removed under reduced pressure. Then, the reaction mixture was separated and purified by MPLC to give (3S,5R,10S,13R,14R,17R)-4,4,10,13,14-pentamethyl-17-((1S)-1-(3-(2-methylprop-1-en-1-yl)oxirane-2-yl)ethyl)-2,3,4,5,6,7, 10,11,12,13,14,15,16,17-tetradecahydro-1H-cyclopenta[a]phenanthrene-3-yl acetate (10 mg, 0.021 mmol) in a yield of 4%.

¹H NMR (400 MHz, CDCl₃) : δ5.33 - 5.25 (m, 1H), 4.50 (dd, *J* = 11.7, 4.6 Hz, 1H), 3.21 - 3.06 (m, 2H), 2.05 - 2.00 (m, 6H), 1.75 (s, 3H), 1.63 (s, 3H), 1.09 (s, 3H), 1.08 (s, 3H), 1.00 (s, 3H), 0.90 (s, 3H), 0.88 (s, 3H), 0.71 (s, 3H).

Mass (GCMS) : 482 (M⁺).

### Step 9: Preparation of (3S,5R,10S,13R,14R,17R)-17-((2S,3S)-3-hydroxy-6-methylhept-5-en-2-yl)-4,4,10,13,14-pentamethyl-2,3,4,5,6,7,10,11,12,13,14,15,16,17-tetradecahydro-1H-cyclopenta[a]phenanthrene-3-yl acetate

The (3S,5R,10S,13R,14R,17R)-4,4,10,13,14-pentamethyl-17-((1S)-1-(3-(2-methylprop-1-en-1-yl)oxirane-2-yl)ethyl)-2,3,4,5,6,7, 10,11,12,13,14,15,16,17-tetradecahydro-1H-cyclopenta[a]phenanthrene-3-yl acetate (10 mg, 0.021 mmol, 1 eq) synthesized in step 8 was dissolved in dichloromethane (50 µl) and tetrahydrofuran (THF) (5 µl), and zinc (II) chloride (8.47 mg, 0.062 mmol, 3 eq) and sodium cyanoborohydride (3.91 mg, 0.062 mmol, 3 eq) were added thereto. Zinc(II) chloride (8.47 mg, 0.062 mmol, 3 eq) and sodium cyanoborohydride (3.91 mg, 0.062 mmol, 3 eq) were added to the reaction mixture, and the mixture was stirred at room temperature for 12 hours. Upon completion of the reaction, extraction was performed with dichloromethane. The organic layer was washed sequentially with H₂O and brine, dried over anhydrous Na₂SO₄, and the solvent was removed under reduced pressure. Then, the reaction mixture was separated and purified by MPLC to give (3S,5R,10S,13R,14R,17R)-17-((2S,3S)-3-hydroxy-6-methylhept-5-en-2-yl)-4,4,10,13,14-pentamethyl-2,3,4,5,6,7,10,11,12,13,14,15,16,17-tetradecahydro-1H-cyclopenta[a]phenanthrene-3-yl acetate (4 mg, 8.46 µmol) in a yield of 40%.

¹H NMR (400 MHz, CDCl₃) : δ5.15 (t, *J* = 7.1 Hz, 1H), 4.50 (dd, *J* = 11.6, 4.5 Hz, 1H), 3.67 (dd, *J* = 8.0, 4.5 Hz, 1H), 2.06 - 1.97 (m, 6H) 1.76 - 1.70 (m, 3H), 1.65 - 1.62 (m, 3H), 1.58 (s, 3H), 1.25 (s, 3H), 1.01 (s, 3H), 0.90 (s, 3H), 0.88 (s, 3H), 0.70 (s, 3H).

Mass (GCMS) : 484 (M⁺).

### Step 10: Preparation of inotodiol

The (3S,5R,10S,13R,14R,17R)-17-((2S,3S)-3-hydroxy-6-methylhept-5-en-2-yl)-4,4,10,13,14-pentamethyl-2,3,4,5,6,7,10,11,12,13,14,15,16,17-tetradecahydro-1H-cyclopenta[a]phenanthrene-3-yl acetate (4 mg, 8.46 µmol) synthesized in step 9 was dissolved in pyridine (100 µl) at 0°C, and an excess of methanesulfonyl chloride (50 µl) was added thereto. The reaction mixture was stirred at 0°C for 5 hours. Upon completion of the reaction, ice was added to the reaction mixture, followed by extraction with diethyl ether. The organic layer was washed sequentially with H₂O and brine, dried over anhydrous Na₂SO₄, and the solvent was removed under reduced pressure. Then, (3S,5R,10S,13R,14R,17R)-4,4,10,13,14-pentamethyl-17-((2S,3S)-6-methyl-3-((methylsulfonyl)oxy)hept-5-en-2-yl)-2,3,4,5,6,7,10,11,12,13,14,15,16,17-tetradecahydro-1H-cyclopenta[a]phenanthrene-3-yl acetate (5 mg) was obtained without further purification.

Thereafter, the (3 S, 5 R, 10 S, 1 3 R, 14 R, 17 R) -4,4, 1 0,1 3,14 - pe nta met hyl-1 7-((2S,3S)-6-methyl-3-((methylsulfonyl)oxy)hept-5-en-2-yl)-2,3,4,5,6,7,10,11,12,13,14,15,16,17-tetradecahydro-1H-cyclopenta[a]phenanthrene-3-yl acetate (5 mg) synthesized above and 18-crown-6 (7.66 mg, 0.029 mmol, 3.4 eq) were dissolved in dimethyl sulfoxide (DMSO) (50 µl) and dimethylformamide (DMF) (50 µl), to which potassium peroxide (2.71 mg, 0.038 mmol, 4.5 eq) was added. The reaction mixture was stirred at room temperature for 5 hours under a nitrogen stream. Potassium peroxide (17 mg, 0.24 mmol, 24 eq) and 18-crown-6 (23 mg, 0.087 mmol, 9 eq) were added to the reaction mixture, followed by stirring at room temperature for 12 hours under a nitrogen stream. Upon completion of the reaction, a few drops of 1 N HCl were added to the reaction mixture, and the mixture was extracted with dichloromethane. The organic layer was washed sequentially with H₂O and brine, dried over anhydrous Na₂SO₄, and the solvent was removed under reduced pressure. Then, the reaction mixture was separated and purified by MPLC to give inotodiol (0.6 mg, 1.35 µmol) in a yield of 16%.

¹H NMR (400 MHz, CDCl₃) : δ5.22 - 5.14 (m, 1H), 3.70 - 3.63 (m, 1H), 3.24 (dd, *J* = 11.1, 4.3 Hz, 1H), 2.04 (dd, *J* = 21.3, 14.3 Hz, 6H), 1.75 (s, 3H), 1.66 (s, 3H), 1.00 (s, 3H), 0.98 (s, 3H), 0.95 (s, 3H), 0.93 (s, 3H), 0.87 (s, 3H), 0.81 (s, 3H), 0.72 (s, 3H).

Mass (GCMS) : 442 (M⁺).

### Example 2: Preparation of inotodiol 2 (corresponding to reaction formulas 1 and 4)

### 2-1. Preparation of inotodiol synthesis precursor (reaction formula 4)

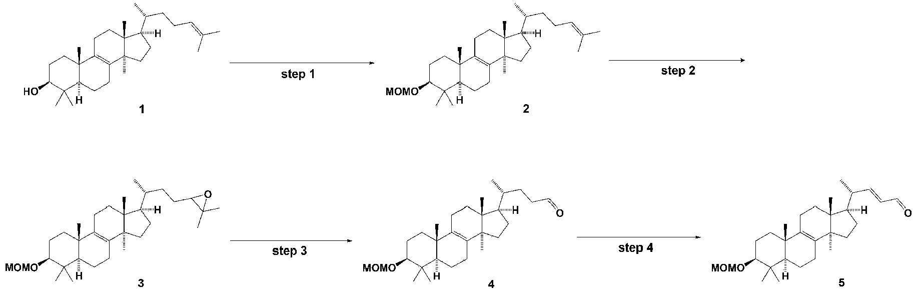

### Step 1: Preparation of (3S,5R,10S,13R,14R,17R)-3-(methoxymethoxy)-4,4,10, 13, 14-pentamethyl-17-((R)-6-methylhept-5-en-2-yl)-2,3,4,5,6,7,10,11,12,13,14,15,16,17-tetradecahydro-1H-cyclopenta[a]phenanthrene

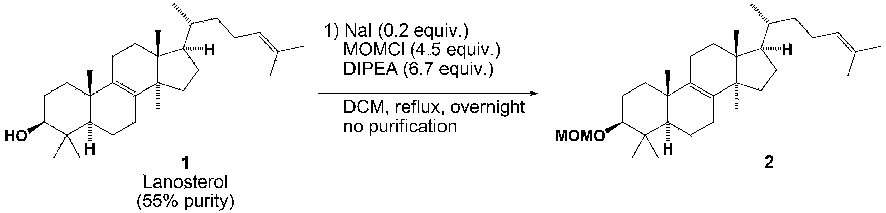

Lanosterol (4 g, 5.91 mmol, 1 eq, purity 55%) and sodium iodide (Nal) (0.089 g, 0.591 mmol, 10 mol %) were dissolved in dichloromethane (50 ml), to which chloromethyl methyl ether (MOM chloride, MOMCI) (1.346 ml, 17.72 mmol, 3 eq) was added. N,N-diisopropylethylamine (4.73 ml, 27.2 mmol, 4.6 eq) was slowly added to the reaction mixture at 0°C. The reaction mixture was stirred under reflux at 50°C for 12 hours. After the reaction mixture was cooled to 0°C, sodium iodide (Nal) (0.044 g, 0.295 mmol, 5 mol %), chloromethyl methyl ether (MOM chloride, MOMCI) (0.673 ml, 8.86 mmol, 1.5 eq), and N,N-diisopropylethylamine (2.366 ml, 13.58 mmol, 2.2 eq) were added to the mixture at the same temperature, and the reaction mixture was stirred under reflux at 50°C for 2 hours. Upon completion of the reaction, a saturated aqueous NaHCO₃ solution was added to the reaction mixture, followed by extraction with dichloromethane. The organic layer was washed sequentially with H₂O and brine, dried over anhydrous Na₂SO₄, and the solvent was removed under reduced pressure. Then, (3S,5R,10S,13R,14R,17R)-3-(methoxymethoxy)-4,4,10,13,14-pentamethyl-17-((R)-6-methylhept-5-en-2-yl)-2,3,4,5,6,7,10,11,12,13,14,15,16,17-tetradecahydro-1H-cyclopenta[a]phenanthrene was obtained without further purification.

¹H NMR (400 MHz, CDCl₃) δ 4.75 (d, *J* = 6.8 Hz, 1H), 4.61 (d, *J* = 6.7 Hz, 1H), 3.39 (s, 3H), 3.15 - 3.07 (m, 1H), 2.02 (m, 6H), 1.68 (s, 3H), 1.60 (s, 3H), 0.99 (d, *J* = 2.0 Hz, 6H), 0.87 (s, 6H), 0.84 (s, 3H), 0.69 (s, 3H).

Mass (GCMS) : 470 (M⁺).

### Step 2: Preparation of 3-((R)-3-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)butyl)-2,2-dimethyloxirane

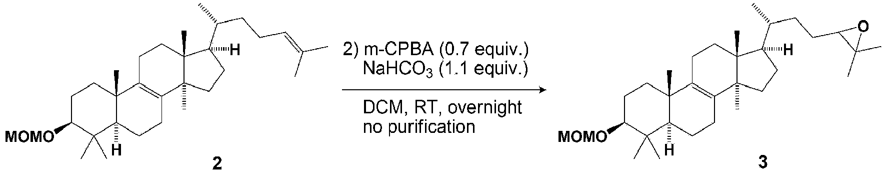

The (3S,5R,10S,13R,14R,17R)-3-(methoxymethoxy)-4,4,10,13,14-pentamethyl-17-((R)-6-methylhept-5-en-2-yl)-2,3,4,5,6,7,10,11,12,13,14,15,16,17-tetradecahydro-1H-cyclopenta[a]phenanthrene (5.195 g, 11.04 mmol, 1 eq) synthesized in step 1 was dissolved in dichloromethane (50 ml), to which meta-chloroperoxybenzoic acid (m-CPBA) (1.904 g, 7.72 mmol, 0.7 eq) and sodium hydrogen carbonate (NaHCO₃) (0.983 g, 11.70 mmol, 1.06 eq) were added at 0°C. The reaction mixture was stirred at room temperature for 12 hours. Upon completion of the reaction, the reaction mixture was filtered, and the filtrate was washed with 10% aqueous NaHCO₃ solution and brine. The reaction mixture was dried over anhydrous MgSO₄ and the solvent was removed under reduced pressure. Then, 3-((R)-3-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)butyl)-2,2-dimethyloxirane was obtained without further purification.

¹H NMR (400 MHz, CDCl₃) δ 4.75 (d, *J* = 6.8 Hz, 1H), 4.61 (d, *J* = 6.7 Hz, 1H), 3.39 (s, 3H), 3.11 (dd, *J* = 11.7, 4.0 Hz, 1H), 2.69 (s, 1H), 2.02 (s, 3H), 1.96 - 1.85 (m, 2H), 1.75 (s, 1H), 1.71 (s, 1H), 1.31 (s, 3H), 1.26 (d, *J* = 4.5 Hz, 4H), 0.99 (d, *J* = 1.7 Hz, 6H), 0.93 - 0.89 (m, 3H), 0.87 (s, 3H), 0.84 (s, 3H), 0.69 (d, *J* = 3.4 Hz, 3H).

Mass (GCMS) : 486 (M⁺).

### Step 3: Preparation of (R)-4-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)pentanal

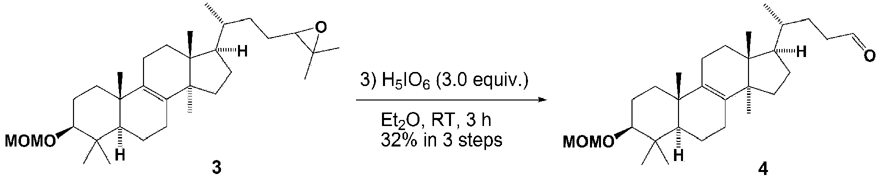

The 3-((R)-3-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)butyl)-2,2-dimethyloxirane (4.55 g, 9.35 mmol, 1 eq) synthesized in step 2 was dissolved in diethyl ether (50 ml), to which periodic acid (6.39 g, 28.0 mmol, 3 eq) was added. The reaction mixture was stirred at room temperature for 3 hours under a nitrogen stream. Upon completion of the reaction, extraction was performed with diethyl ether. The organic layer was washed with H₂O, dried over anhydrous MgSO₄, and the solvent was removed under reduced pressure. Then, the reaction mixture was separated and purified by MPLC to give (R)-4-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)pentanal (1.327 g, 2.98 mmol) as a white solid in a yield of 32%.

¹H NMR (400 MHz, CDCl₃) δ 9.77 (t, *J* = 1.8 Hz, 1H), 4.75 (d, *J* = 6.7 Hz, 1H), 4.61 (d, *J* = 6.7 Hz, 1H), 3.39 (s, 3H), 3.11 (dd, *J* = 11.7, 4.2 Hz, 2H), 2.02 (s, 6H), 0.99 (s, 6H), 0.90 (d, *J* = 6.0 Hz, 3H), 0.87 (s, 3H), 0.84 (s, 3H).

Mass (GCMS) : 444 (M⁺).

### Step 4: Preparation of (R,E)-4-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)pent-2-enal

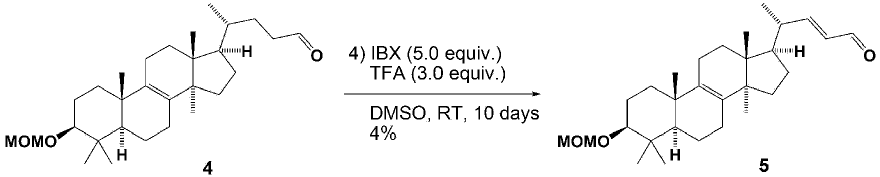

The (R)-4-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)pentanal (0.792 g, 1.781 mmol, 1 eq) synthesized in step 3 was dissolved in dimethyl sulfoxide (DMSO) (50 ml), to which 2-iodobenzoic acid (IBX) (997 mg, 3.56 mmol, 2 eq) and trifluoroacetic acid (TFA) (0.048ml, 0.623 mmol, 35 mol%) were added. The reaction mixture was stirred at room temperature for 12 hours. 2-lodobenzoic acid (IBX) (498 mg, 1.78 mmol, 1 eq) and trifluoroacetic acid (TFA) (0.14 ml, 1.78 mmol, 1 eq) were added to the reaction mixture, and the mixture was stirred at room temperature for 9 hours. Monitoring was conducted until the reaction was terminated. Upon completion of the reaction, a saturated aqueous NaHCO₃ solution was added, followed by extraction with ethyl acetate (EA). The organic layer was washed sequentially with H₂O and brine, dried over anhydrous Na₂SO₄, and filtered with celite. The solvent was removed under reduced pressure, and after completion of the reaction, a saturated aqueous NaHCO₃ solution was added, followed by extraction with dichloromethane. The organic layer was washed sequentially with H₂O and brine, dried over anhydrous Na₂SO₄, and the solvent was removed under reduced pressure. The reaction mixture was separated and purified by MPLC to give (R,E)-4-((3S,5R,10S,13R,14R,17R)-3-(methoxymethoxy)-4,4,10,13,14-pentamethyl-2,3,4,5,6,7,10,11,12,13,14,15,16,17-tetradecahydro-1H-cyclopenta[a]phenanthrene-17-yl)pent-2-enal (32 mg, 0.072 mmol) as a yellow solid in a yield of 4%.

¹H NMR (400 MHz, CDCl₃) δ 9.49 (d, *J* = 7.9 Hz, 1H), 6.72 (dd, *J* = 15.5, 8.4 Hz, 1H), 6.06 (dd, *J* = 15.5, 7.5 Hz, 1H), 4.75 (d, *J* = 6.7 Hz, 1H), 4.62 (d, *J* = 6.6 Hz, 1H), 3.39 (s, 3H), 3.11 (dd, *J* = 11.5, 3.9 Hz, 1H), 2.06 - 1.96 (m, 3H), 1.00 (d, *J* = 4.3 Hz, 6H), 0.87 (t, *J* = 5.5 Hz, 8H), 0.84 (s, 3H), 0.75 (s, 1H), 0.69 (s, 1H).

Mass (GCMS) : 442 (M⁺).

Thereafter, inotodiol was prepared through the same process using the compound of formula 5 prepared in step 4 as a starting material of step 7 of <Example 1> described above.

As mentioned above, the present invention has been described in detail through the preferred preparative examples, examples and experimental examples, but the scope of the present invention is not limited to the specific examples, and should be interpreted by the appended claims. In addition, those of ordinary skill in the art should understand that many modifications and variations are possible without departing from the scope of the present invention.

## Claims

1. A method for preparing a compound represented by formula 1 comprising the following steps, as shown in reaction formula 1 below:
step 1 of preparing a compound represented by formula 9 by epoxization of a compound represented by formula 8;
step 2 of preparing a compound represented by formula 10 by reacting the compound represented by formula 9;
step 3 of preparing a compound represented by formula 11 by reacting the compound represented by formula 10; and
step 4 of preparing a compound represented by formula 1 by reacting the compound represented by formula 11: 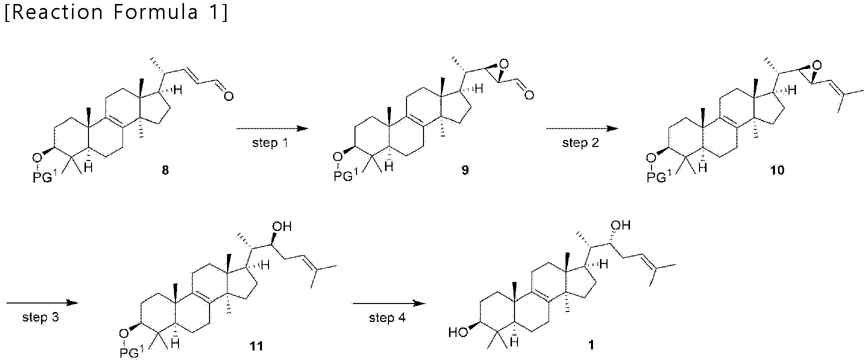 In reaction formula 1 above,
PG¹ is a protecting group of an alcohol.

2. The method according to claim 1, wherein the PG¹ is a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2-(trimethylsilyl)ethoxymethyl (SEM) and (phenyldimethylsilyl)methoxymethyl (SMOM).

3. The method according to claim 1, wherein the compound represented by formula 8 is prepared through a preparation method comprising the following steps, as shown in reaction formula 2 below:
step 1 of preparing a compound represented by formula 7 by epoxization of a compound represented by formula 6; and
step 2 of preparing a compound represented by formula 8 by reacting the compound represented by formula 7: 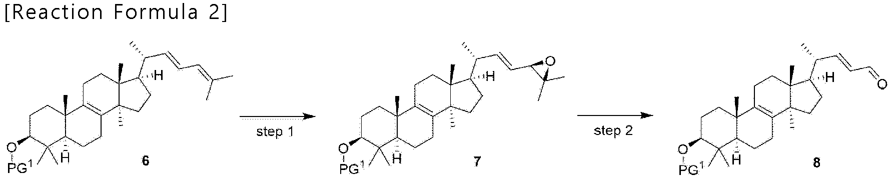 In reaction formula 2 above,
PG¹ is a protecting group of an alcohol.

4. The method according to claim 3, wherein the compound represented by formula 6 is prepared through a preparation method comprising the following steps, as shown in reaction formula 3 below:
step 1 of preparing a compound represented by formula 3 by reacting a compound represented by formula 2;
step 2 of preparing a compound represented by formula 4 by elimination reaction of the compound represented by formula 3;
step 3 of preparing a compound represented by formula 5 by elimination reaction of the compound represented by formula 4; and
step 4 of preparing a compound represented by formula 6 by reacting the compound represented by formula 5: 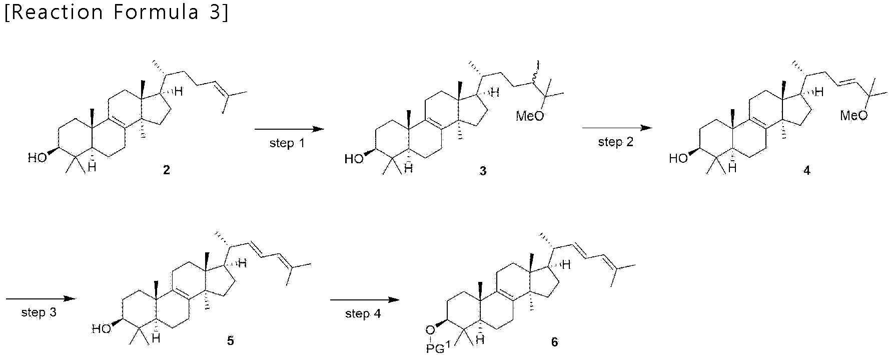 In reaction formula 3 above,
PG¹ is a protecting group of an alcohol.

5. The method according to claim 3 or 4, wherein the PG¹ is a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2-(trimethylsilyl)ethoxymethyl (SEM) and (phenyldimethylsilyl)methoxymethyl (SMOM).

6. A compound represented by formula 8 of claim 1, an isomer thereof, a solvate thereof, or a salt thereof.

7. A compound represented by formula 11 of claim 1, an isomer thereof, a solvate thereof, or a salt thereof.

8. A compound represented by formula 10 of claim 1, an isomer thereof, a solvate thereof, or a salt thereof.

9. A compound represented by formula 9 of claim 1, an isomer thereof, a solvate thereof, or a salt thereof.

10. A compound represented by formula 7 of claim 3, an isomer thereof, a solvate thereof, or a salt thereof.

11. The method according to claim 1, wherein the compound represented by formula 8 is prepared through a preparation method comprising the following steps, as shown in reaction formula 4 below:
step 1 of preparing a compound represented by formula 2' by introducing a protecting group to a compound represented by formula 1';
step 2 of preparing a compound represented by formula 3' by epoxization of the compound represented by formula 2' prepared in step 1 above;
step 3 of preparing a compound represented by formula 4' by reacting the compound represented by formula 3' prepared in step 2 above; and
step 4 of preparing a compound represented by formula 8 by reacting the compound represented by formula 4' prepared in step 3 above: 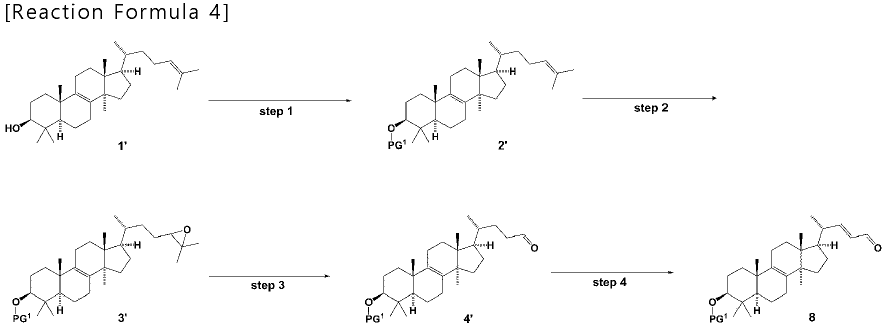 In reaction formula 4 above,
PG¹ is a protecting group of an alcohol.

12. The method according to claim 11, wherein the PG¹ is a protecting group of one alcohol selected from the group consisting of acetyl (Ac), benzyl (Bn), methoxymethyl (MOM), 2-methoxyethoxymethyl (MEM), methylthiomethyl (MTM), tetrahydropyranyl (THP), benzyloxymethyl (BOM), p-methoxyphenyl (PMP), p-methoxybenzyl (PMB), p-methoxybenzyloxymethyl (PMBM), triisopropylsilyl (TIPS), tert-butyldimethylsilyl (TBDMS), 2-(trimethylsilyl)ethoxymethyl (SEM) and (phenyldimethylsilyl)methoxymethyl (SMOM).

13. A compound represented by formula 3' of claim 11, an isomer thereof, a solvate thereof, or a salt thereof.

14. A compound represented by formula 4' of claim 11, an isomer thereof, a solvate thereof, or a salt thereof.

15. The compound, the isomer thereof, the solvate thereof, or the salt thereof according to claim 13 or 14, wherein the isomer is a stereoisomer.

16. The compound, the isomer thereof, the solvate thereof, or the salt thereof according to claim 15, wherein the stereoisomer is an optical isomer.

17. The compound, the isomer thereof, the solvate thereof, or the salt thereof according to claim 13 or 14, wherein the salt is a pharmaceutically acceptable salt.
